# EUROPEAN PATENT SPECIFICATION

(11) **EP 4 489 645 B1**
(45) Date of publication and mention of the grant of the patent: **21.05.2025**
(21) Application number: 23718475.9
(22) Date of filing: 27.03.2023
(51) Int. Cl.: A61B 5/15, A61B 5/153, A61B 5/154, A61M 39/10

(54) **HEMOLYSIS-REDUCTION CONNECTOR FOR DIRECT BLOOD DRAW**
HÄMOLYSEREDUKTIONSVERBINDER FÜR DIREKTE BLUTENTNAHME
CONNECTEUR DE RÉDUCTION D'HÉMOLYSE POUR PRÉLÈVEMENT DIRECT DE SANG

(30) Priority: 29.03.2022 US 202263324991 P
(43) Date of publication of application: 15.01.2025
(73) Proprietor: CareFusion 303, Inc., San Diego, CA 92130 (US)
(72) Inventor: SALEHI BOROJERDI, Alireza, San Diego, California 92130 (US); JADHAV, Amarsinh Deeliprao, Karnataka Bengaluru 560045 (IN); KHAN, Mohammed Mehtab, Karnataka Bengaluru 560045 (IN); BIERITZ, Shelby Ann, San Diego, California 92130 (US)
(74) Representative: Zenz Patentanwälte Partnerschaft mbB
(86) International application number: PCT/US2023/016439
(87) International publication number: WO 2023/192193

(56) References cited:
- WO-A1-2021/026223
- CN-U- 214 485 271
- US-A1- 2015 025 348
- US-A1- 2021 137 436
- US-A1- 2021 228 127

## Description

### TECHNICAL FIELD

The present disclosure generally relates to blood draw and administration of parenteral fluids to a patient, and particularly to systems and non claimed methods to reduce hemolysis in PIVC blood draw using a connector.

### BACKGROUND

Catheters are commonly used for a variety of infusion therapies. For example, catheters may be used for infusing fluids, such as normal saline solution, various medicaments, and total parenteral nutrition, into a patient. Catheters may also be used for withdrawing blood from the patient.

A common type of catheter is an over-the-needle peripheral intravenous ("IV") catheter (PIVC). As its name implies, the over-the-needle catheter may be mounted over an introducer needle having a sharp distal tip. A catheter assembly may include a catheter hub, the catheter extending distally from the catheter hub, and the introducer needle extending through the catheter. The catheter and the introducer needle may be assembled so that the distal tip of the introducer needle extends beyond the distal tip of the catheter with the bevel of the needle facing up away from skin of the patient. The catheter and introducer needle are generally inserted at a shallow angle through the skin into vasculature of the patient.

In order to verify proper placement of the introducer needle and/or the catheter in the blood vessel, a clinician generally confirms that there is "flashback" of blood in a flashback chamber of the catheter assembly. Once placement of the needle has been confirmed, the clinician may temporarily occlude flow in the vasculature and remove the needle, leaving the catheter in place for future blood withdrawal or fluid infusion.

For blood withdrawal or collecting a blood sample from a patient, a blood collection container may be used. The blood collection container may include a syringe. Alternatively, the blood collection container may include a test tube with a rubber stopper at one end. In some instances, the test tube has had all or a portion of air removed from the test tube so pressure within the test tube is lower than ambient pressure. Such a blood collection container is often referred to as an internal vacuum or a vacuum tube. A commonly used blood collection container is a VACUTAINER^{®} blood collection tube, available from Becton Dickinson & Company.

The blood collection container may be coupled to the catheter. When the blood collection container is coupled to the catheter, a pressure in the vein is higher than a pressure in the blood collection container, which pushes blood into the blood collection container, thus filling the blood collection container with blood. A vacuum within the blood collection container decreases as the blood collection container fills, until the pressure in the blood collection container equalizes with the pressure in the vein, and the flow of blood stops.

Unfortunately, as blood is drawn into the blood collection container, red blood cells are in a high shear stress state and susceptible to hemolysis due to a high initial pressure differential between the vein and the blood collection container. Hemolysis may result in rejection and discard of a blood sample. The high initial pressure differential can also result in catheter tip collapse, vein collapse, or other complications that prevent or restrict blood from filling the blood collection container. Furthermore, blood spillage during and/or after blood draw commonly occurs.

The description provided in the background section should not be assumed to be prior art merely because it is mentioned in or associated with the background section. The background section may include information that describes one or more aspects of the subject technology. US 2021/228127 A1 discloses an adapter including a distal end, which may be configured to couple to a catheter assembly. The adapter may include a proximal end, which may include a proximal connector configured to couple to a blood collection device. The adapter may include a fluid pathway disposed between the distal end and the proximal end, wherein the fluid pathway includes a non-linear portion. The non-linear portion may form a coil shape, an S-shape, or another suitable shape.

### SUMMARY

The invention is defined in the independent claim 1.

Preferable embodiments are further laid out in the dependent claims. The present disclosure provides a flow restriction device, comprising: a housing defining a first lumen, a second lumen, and a cavity disposed between the first lumen and the second lumen; and an insert body disposed within the cavity, the insert body comprising: a first end; a second end; a longitudinal axis extending through the first and second ends; an outer surface; and a channel defined on the outer surface, wherein the channel extends between the first and second ends, the channel comprising a first portion that extends in a first direction away from the longitudinal axis and a second portion that extends in a second direction toward the longitudinal axis, and the channel and an inner surface of the cavity define a fluid passage in fluid communication with the first lumen and the second lumen.

In some instances, the present disclosure provides a flow restriction device, comprising: a male luer connector portion defining a first lumen; a female luer connector portion defining a second lumen, wherein the male luer portion connector and the female luer connector portion cooperatively define a cavity; and an insert body disposed within the cavity, the insert body comprising: a first end; a second end; an outer surface; and a serpentine channel defined on the outer surface, wherein the channel extends between the first and second ends and the channel and an inner surface of the cavity define a fluid passage in fluid communication with the first lumen and the second lumen.

In some aspects, the present disclosure provides a peripheral intravenous catheter assembly configured to limit hemolysis during drawing of blood from a patient, comprising: a catheter hub having a proximal end and a distal end; a fluid collection device; and a flow restriction device, comprising: a housing defining a first lumen, a second lumen, and a cavity disposed between the first lumen and the second lumen, wherein the first lumen is in fluid communication with the catheter hub and the second lumen is in fluid communication with the fluid collection device; and an insert body disposed within the cavity, the insert body comprising: a first end; a second end; an outer surface; and a tortuous channel defined on the outer surface, wherein the channel extends between the first and second ends and the channel and an inner surface of the cavity define a fluid passage in fluid communication with the first lumen and the second lumen.

### BRIEF DESCRIPTION OF THE DRAWINGS

The following figures are included to illustrate certain aspects of the embodiments, and should not be viewed as exclusive embodiments.
FIG. 1 illustrates a vascular access device including a peripheral intravenous catheter (PIVC) assembly that includes a flow restriction device, in accordance with some embodiments of the present disclosure.
FIG. 2 illustrates a perspective view of the flow restriction device, in accordance with some embodiments of the present disclosure.
FIG. 3 illustrates an exploded view of the flow restriction device of FIG. 2, in accordance with some embodiments of the present disclosure.
FIG. 4 illustrates a cross-sectional view of the flow restriction device of FIG. 2, in accordance with some embodiments of the present disclosure.

### DETAILED DESCRIPTION

The detailed description set forth below describes various configurations of the subject technology and is not intended to represent the only configurations in which the subject technology may be practiced. The detailed description includes specific details for the purpose of providing a thorough understanding of the subject technology. Accordingly, dimensions may be provided in regard to certain aspects as non-limiting examples. However, it will be apparent to those skilled in the art that the subject technology may be practiced without these specific details. In some instances, well-known structures and components are shown in block diagram form in order to avoid obscuring the concepts of the subject technology.

Various aspects of the subject technology will now be disclosed according to particular but non-limiting examples. Various embodiments described in the present disclosure may be carried out in different ways and variations, and in accordance with a desired application or implementation.

Blood draw via a vascular access device has drawn increasing attention attributed to minimized needle sticks and improved operation efficiency as compared with traditional blood draw methods with venipuncture. Current blood draw using a peripheral intravenous catheter (PIVC) has seen some challenges, one of the most critical is hemolysis related blood quality. In particular, with currently existing PIVC products in the market, along with the standard connection (such as a short extension set and a needleless connector), and blood collection devices (such as a Vacutainer), the shear stress exerted onto blood cells tends to be on the verge of hemolyzing.

Various embodiments of the present disclosure are directed to providing systems and non claimed methods to address hemolysis in PIVC blood draw with a hemolysis reduction accessory (also referred to herein as a flow restriction device), such as a connector, which is attached to the PIVC and serves as a flow restrictor to reduce risk of hemolysis. The hemolysis-reduction accessory is advantageously compatible with PIVC placement and does not necessitate change to any of the existing operations. The hemolysis-reduction accessory of the various embodiments described herein is potentially applicable to a wide variety of PIVC products, and compatible with existing blood collection devices and infusion disposables.

Various embodiments of the present disclosure focus on effective flow restriction with the add-on hemolysis-reduction accessory (also referred to herein as a flow restriction device) that regulates the overall flow rate of the entire fluid path as blood cells travel through. The flow restriction device can be either assembled with the PIVC or co-packaged with the PIVC. The clinician may connect a blood collection device to the port of the accessory and can then draw blood to the intended volume. After blood draw, the clinician may disconnect and discard the flow restriction device and the blood collection device together. As such, this flow restriction device can be either for single blood draw or stay inline throughout indwell.

The flow restriction devices and associated blood collection systems of the various embodiments described herein additionally provide further advantages over currently existing blood collection systems. For example, add-on flow restriction devices described herein allow for hemolysis-reduction function to be integrated for PIVC blood draw. Further, the flow restriction devices described herein are compatible with PIVC placement and allow for seamless blood draw at insertion. Additionally, since the flow restriction devices are an add-on which can be easily incorporated without any changes to existing PIVC, there is minimal impact to clinical setting and operations.

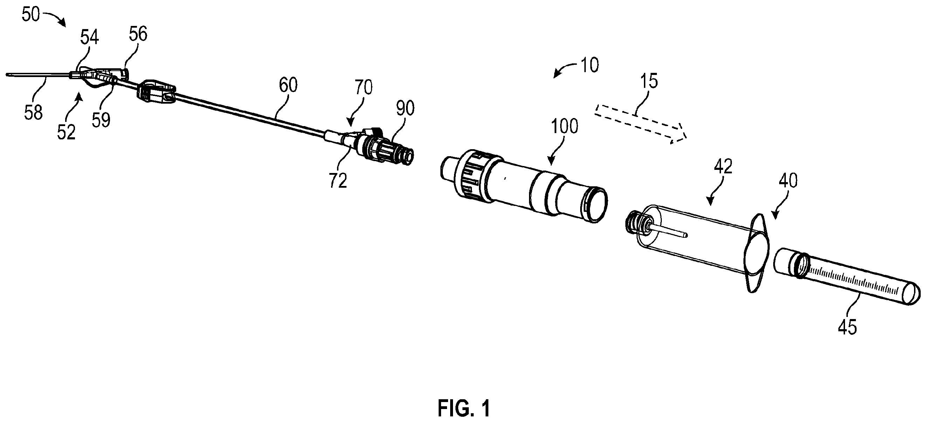

FIG. 1 illustrates a vascular access device 10 including a peripheral intravenous catheter (PIVC) assembly 50 that includes a connector or flow restriction device 100, in accordance with some embodiments of the present disclosure. The flow restriction device 100 may be configured to facilitate connections for components of the vascular access device 10 and reduce a likelihood of hemolysis during blood collection using the vascular access device 10. In some embodiments, the vascular access device 10 may include a catheter assembly 50. The catheter assembly 50 may include a catheter hub 52, which may include a distal end 54, a proximal end 56, and a lumen extending through the distal end and the proximal end. The catheter assembly 50 may further include a catheter 58, which may be secured within the catheter hub 52 and may extend distally from the distal end 54 of the catheter hub 52. In some embodiments, the catheter assembly 50 may be a peripheral intravenous catheter (PIVC).

In some embodiments, the catheter assembly 50 may include or correspond to any suitable catheter assembly 50. In some embodiments, the catheter assembly 50 may be integrated and include an extension tube 60, which may extend from and be integrated with a side port 59 of the catheter hub 52. A non-limiting example of an integrated catheter assembly is the BD NEXIVA^{™} Closed IV Catheter system, available from Becton Dickinson and Company. In some embodiments, a proximal end of the extension tube 60 may be coupled to an adapter, such as, for example, a Y-adapter 70. In some embodiments, the flow restriction device 100 may be fluidly coupled to the Y-adapter 70.

In some embodiments, the catheter assembly 50 may be non-integrated and may not include the extension tube 60. In these and other embodiments, the flow restriction device 100 may be configured to couple to the proximal end 56 of the catheter hub 52 or another suitable portion of the catheter assembly 50. In some embodiments, the flow restriction device 100 may be coupled directly to the catheter assembly 50, eliminating the extension tube 60 and providing a compact catheter system.

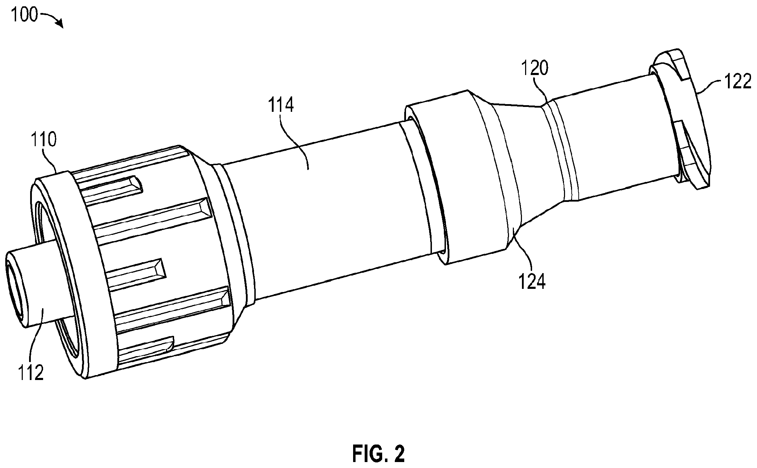

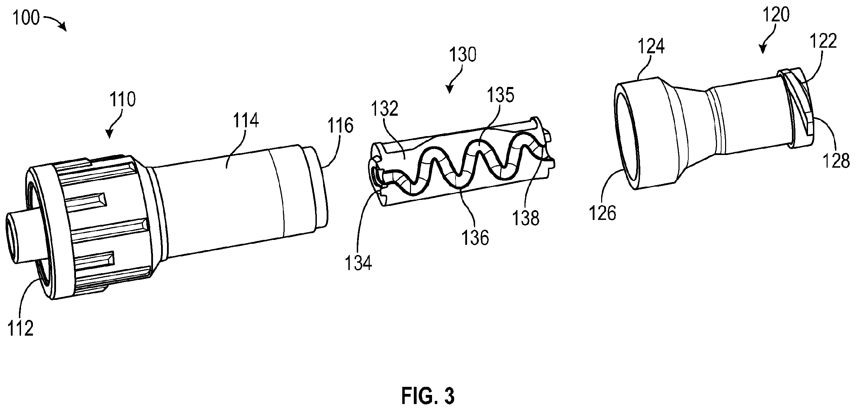

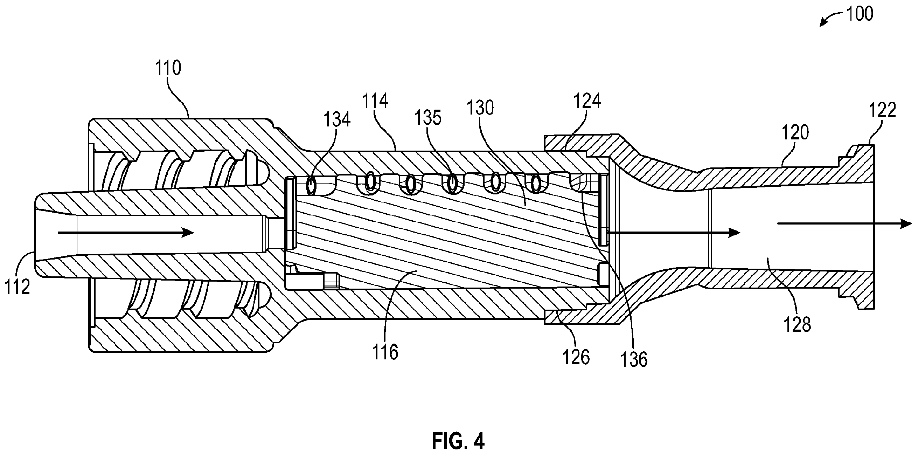

FIG. 2 illustrates a perspective view of the flow restriction device 100, in accordance with some embodiments of the present disclosure. FIG. 3 illustrates an exploded view of the flow restriction device 100 of FIG. 2, in accordance with some embodiments of the present disclosure. FIG. 4 illustrates a cross-sectional view of the flow restriction device 100 of FIG. 2, in accordance with some embodiments of the present disclosure.

As illustrated in FIGS. 2-4, and with continued reference to FIG. 1, in some embodiments, the flow restriction device 100 may include a male luer connector portion 110 configured to couple to the catheter assembly 50. The male luer connector portion 110 may have an internal surface defining a lumen 112 of the male luer connector portion 110. In some embodiments, a distal end of the male luer connector portion 110 can be coupled to the catheter assembly 50. In some embodiments, the lumen 112 is in fluid communication with a cavity 116 defined by the body 114 of the male luer connector portion 110.

In some embodiments, the flow restriction device 100 may further include a female luer connector portion 120 disposed proximally to the male luer connector portion 110. The female luer connector portion 120 may be configured to couple to fluid collection device 40 (e.g., a blood collection device). For example, the female luer connector portion 120 may be integrated with the blood collection device 40 or monolithically formed with the blood collection device 40 as a single unit or piece. As another example, the female luer connector portion 120 may be in the form of a female luer connector or another suitable connector, which may be coupled with a male luer portion of the blood collection device 40. The female luer connector portion 120 may include an internal surface defining a lumen 122 extending therethrough for coupling to the male luer portion of the blood collection device 40. In some embodiments, a proximal end of the female luer connector portion 120 can be coupled to the blood collection device 40. Optionally, a threaded portion 128 of the female luer connector portion 120 can be utilized to engage the female luer connector portion 120 to a corresponding male luer portion. In some embodiments, the lumen 122 is in fluid communication with a cavity 126 defined by the body 124 of the female luer connector portion 120.

In the depicted example, the body 114 of the male luer connector portion 110 can be coupled to the body 124 of the female luer connector portion 120 to allow fluid communication between the lumen 112 and the lumen 122 of the flow restriction device 100 and form a common housing. As illustrated, the cavity 116 of the male luer connector portion 110 can be placed in fluid communication with the cavity 126 of the female luer connector portion 120 to form a common cavity. Accordingly, the lumen 112 of the male luer connector portion 110 can be in fluid communication with the lumen 122 of the female luer connector portion 120 through the common cavity formed via the respective cavities 116, 126.

In the depicted example, an insert body 130 can be disposed within the common cavity formed by the male luer connector portion 110 and the female luer connector portion 120 to control fluid flow between the lumens 112, 122 and reduce hemolysis through the flow restriction device 100. As illustrated, the insert body 130 directs fluid flow between the lumens 112 and 122 of the male luer connector portion 110 and the female luer connector portion 120, respectively while inducing a resistance to the fluid flow as it moves across the insert body 130. By inducing a resistance to the fluid flow, the rate of fluid flow is reduced as the fluid moves across the insert body 130. Reducing the flow rate of a fluid, such as blood, through the flow restriction device 100 can reduce the hemolysis index of the blood.

In some embodiments, an insert body 130 defines a channel 132 that directs fluid flow from a distal end 134 of the insert body 130 to a proximal end 138 of the insert body 130. As illustrated, the channel 132 can be defined along an outer surface of the insert body 130. Accordingly, when the insert body 130 is positioned within the common cavity defined by the male luer connector portion 110 and the female luer connector portion 120, at least a portion of the outer surface of the insert body 130 can engage against an inner surface of the common cavity to permit the channel 132 and the inner surface of the common cavity to cooperatively define a fluid passage therebetween. The defined fluid passage can extend along a path between the distal end 134 and the proximal end 138 of the insert body 130 to reduce a rate of fluid flow across the insert body 130. In some embodiments, the channel 132 may be defined on an inner surface of the body 114, such that when the insert body 130 is received within the connector portion 110, a pathway is formed by engagement of the outer surface of the insert body 130 and the inner surface of the body 114. In some embodiments, the channel 132 is an open channel on either or both of the insert body 130 and the connector portion 120 body 114 that is closed to form a passageway by the engagement of the inner surface of the body 114 and the outer surface of the insert body 130.

Therefore, in some embodiments, the channel 132 (in conjunction with the inner surface of the common cavity) can direct fluid flow from the male luer connector portion 110 into the fluid collection device 40 via the female luer connector portion 120. As depicted, the channel 132 may fluidly communicate the catheter assembly 50 with the fluid collection device 40 via the flow restriction device 100. For example, in some embodiments, a leg 72 of the Y-adapter 70 may be coupled to the flow restriction device 100. The leg 72 of Y-adapter 70 may include a lumen into which the distal end of the male luer connector portion 110 may be coupled. The Y-adapter 70 may fluidly communicate the flow restriction device 100 via a connector 90, which is depicted as a needleless connector, and the channel 132 with the catheter assembly 50, for example, via the extension tubing 60. Accordingly, the channel 132 may define a fluid pathway with a serpentine, tortuous, extended, or otherwise indirect path (as discussed below) through which fluid entering the flow restriction device 100 from the catheter assembly 50, may flow through the flow restriction device 100 for collection in the fluid collection device 40. For example, where blood is being withdrawn or collected from a patient, the medical fluid may be blood, and the fluid collection device 40 may be a blood collection device. In some embodiments, the blood collection device may be a luer lock access device (LLAD). Accordingly, during blood collection or withdrawal from the patients, the blood sample may flow from the distal end of the male luer connector portion 110 into the LLAD 40 via the flowpath or channel 132.

In some embodiments, the insert body 130 can define a longitudinal axis that extends through the distal and proximal ends 134, 138. The channel 132 spans between the distal and proximal ends 134, 138 of the insert body 130, and is configured to extend in more than one direction along a path between the distal and proximal ends 134, 138. Because the path extends in more than one direction, the channel 132 extends indirectly between the distal and proximal ends 134, 138 of the insert body 130. The indirect path between the distal and proximal ends 134, 138 of the insert body 130 can have a length that is greater than a distance between the distal and proximal ends 134, 138 of the insert body 130.

As illustrated, the channel 132 can be disposed along a serpentine, tortuous, or turning path. In other words, the channel 132 can include a first portion 136 that extends in a first direction away from the longitudinal axis and a second portion 135 that extends in a second direction toward the longitudinal axis. Further, the channel 132 can include additional portions that extend toward and away from the longitudinal axis. The change in direction of the channel 132 between the distal and proximal ends 134, 138 of the insert body 130 can be configured by an angle between one or more portion of the channel 132. In some instances, the channel 132 can have a change in direction by an angle between approximately 45 degrees and approximately 90 degrees. The changes in direction of the channel 132 can form a line that represents a periodic wave, such as a sinusoidal wave. The changes in direction of the channel 132 are optimized to reduce pressure of a fluid, e.g., blood, moving through the channel 132 by inducing resistance to the fluid. Since the decreased blood flow rate causes a reduction in shear stress experienced by the red blood cells of the blood, a risk of hemolysis during blood collection may advantageously be reduced. In some embodiments, the channel 132 can have a diameter in the range of about 0.508 millimeters to about 0.762 millimeters (about 0.02 inches to 0.03 inches). In some embodiments, the length of the channel 132 can be of about 24.639 millimeters to about 33.02 millimeters (about 0.97 inches to about 1.30 inches).

As illustrated, the path of the channel 132 can be disposed or otherwise confined to a portion of the outer surface of the insert body 130. For example, the path of the channel 132 may extend within an arc defined by the cross-section of the cylindrical insert body 130. In some embodiments, the path of the channel 132 can extend within an 180-degree arc defined by the cross-section of the cylindrical insert body 130. In some embodiments, the path of the channel 132 can extend within a 90-degree arc defined by the cross-section of the cylindrical insert body 130.

The flow restriction device 100 of the various embodiments described herein is advantageous over currently existing blood collection systems. For example, during blood draw with currently existing blood draw devices, blood cells may experience wall shear stress as they flow from the distal end to the proximal end of the blood collection systems. Wall shear stress on blood cells is considered a major source of mechanical damage to blood cells causing hemolysis of the blood cells. The diameter and effective length of the channel 132 may facilitate increased flow resistance within the vascular access system to distribute the pressure differential and reduce shear stress experienced by the red blood cells of the blood 15. For example, the minimized diameter and elongated effective length of the channel 132 may provide increased resistance to flow of the blood 15 and thereby decrease blood flow rate within the flow restriction device 100. Since the decreased blood flow rate causes a reduction in shear stress experienced by the red blood cells of the blood 15, a risk of hemolysis during blood collection may advantageously be reduced. Further, the geometry of the channel 132 can reduce the amount of blood wasted by currently existing blood collection systems.

Further, the flow restriction device 100 can reduce hemolysis without the use of active devices such as valves or other flow control devices. In some applications, the configuration of the flow restriction device 100 as described herein can minimize flushing of the vascular access device 10, minimize dead volume, and facilitate a compact design. Further, the configuration of the flow restriction device 100 can facilitate ease of manufacturing compared to certain prior art designs. For example, in some applications, the insert body 130 can be press fit within the male luer connector portion 110 followed by ultrasonic welding of the female luer connector portion 120 to form the flow restriction device 100.

The present disclosure is provided to enable any person skilled in the art to practice the various aspects described herein. The disclosure provides various examples of the subject technology, and the subject technology is not limited to these examples. Various modifications to these aspects will be readily apparent to those skilled in the art, and the generic principles defined herein may be applied to other aspects.

A reference to an element in the singular is not intended to mean "one and only one" unless specifically so stated, but rather "one or more." Unless specifically stated otherwise, the term "some" refers to one or more. Pronouns in the masculine (e.g., his) include the feminine and neuter gender (e.g., her and its) and vice versa. Headings and subheadings, if any, are used for convenience only and do not limit the invention.

The word "exemplary" is used herein to mean "serving as an example or illustration." Any aspect or design described herein as "exemplary" is not necessarily to be construed as preferred or advantageous over other aspects or designs. In one aspect, various alternative configurations and operations described herein may be considered to be at least equivalent.

As used herein, the phrase "at least one of" preceding a series of items, with the term "or" to separate any of the items, modifies the list as a whole, rather than each item of the list. The phrase "at least one of" does not require selection of at least one item; rather, the phrase allows a meaning that includes at least one of any one of the items, and/or at least one of any combination of the items, and/or at least one of each of the items. By way of example, the phrase "at least one of A, B, or C" may refer to: only A, only B, or only C; or any combination of A, B, and C.

A phrase such as an "aspect" does not imply that such aspect is essential to the subject technology or that such aspect applies to all configurations of the subject technology. A disclosure relating to an aspect may apply to all configurations, or one or more configurations. An aspect may provide one or more examples. A phrase such as an aspect may refer to one or more aspects and vice versa. A phrase such as an "embodiment" does not imply that such embodiment is essential to the subject technology or that such embodiment applies to all configurations of the subject technology. A disclosure relating to an embodiment may apply to all embodiments, or one or more embodiments. An embodiment may provide one or more examples. A phrase such an embodiment may refer to one or more embodiments and vice versa. A phrase such as a "configuration" does not imply that such configuration is essential to the subject technology or that such configuration applies to all configurations of the subject technology. A disclosure relating to a configuration may apply to all configurations, or one or more configurations. A configuration may provide one or more examples. A phrase such a configuration may refer to one or more configurations and vice versa.

In one aspect, unless otherwise stated, all measurements, values, ratings, positions, magnitudes, sizes, and other specifications that are set forth in this specification, including in the claims that follow, are approximate, not exact. In one aspect, they are intended to have a reasonable range that is consistent with the functions to which they relate and with what is customary in the art to which they pertain.

## Claims

1. A flow restriction device (100), comprising: a housing defining a first lumen (112), a second lumen (122), and a cavity (116) disposed between the first lumen and the second lumen; and an insert body (130) disposed within the cavity, the insert body comprising: a first end (134); a second end (138); a longitudinal axis extending through the first and second ends; an outer surface; and
a channel (132) defined on the outer surface, wherein the channel extends between the first and second ends, the channel comprising a first portion (136) that extends in a first direction away from the longitudinal axis, when the channel is seen from the top in a direction perpendicular to the longitudinal axis and a second portion (135) that extends in a second direction toward the longitudinal axis, when the channel is seen from the top in a direction perpendicular to the longitudinal axis and the channel and an inner surface of the cavity define a fluid passage in fluid communication with the first lumen and the second lumen,
**characterised in that** the second portion is disposed at an angle between 45 degrees and 90 degrees relative to the first portion.

2. The flow restriction device of Claim 1, wherein the channel has a diameter of about 0.508 millimeters to about 0.762 millimeters (about 0.02 inches to about 0.03 inches).

3. The flow restriction device of Claim 1, wherein the channel has a length of about 24.639 millimeters to about 33.02 millimeters (about 0.97 inches to about 1.30 inches).

4. The flow restriction device of Claim 1, wherein the fluid passage is configured to increase flow resistance through the fluid passage to minimize shear stress experienced by the fluid flow.

5. The flow restriction device of Claim 1, wherein the channel defines a serpentine fluid passage.

6. The flow restriction device of Claim 1, wherein the channel is at least partially sinusoidal.

7. The flow restriction device of Claim 1, wherein the insert body comprises a cylindrical body.

8. The flow restriction device of Claim 7, wherein the channel extends along the outer surface within an arc of a cross-section of the cylindrical body.

9. The flow restriction device of Claim 8, wherein the arc is about 180 degrees.

10. The flow restriction device of Claim 8, wherein the arc is about 90 degrees.

11. The flow restriction device of Claim 1, wherein the housing comprises a male luer connector portion (110) defining the first lumen and a female luer connector portion (120) defining the second lumen, and the male luer portion connector and the female luer connector portion are coupled together to cooperatively define the cavity.

12. The flow restriction device of Claim 1, wherein the first lumen is configured to be coupled to a catheter hub (52).

13. The flow restriction device of Claim 1, wherein the second lumen is configured to coupled to a fluid collection device (40).

14. A peripheral intravenous catheter assembly (10) configured to limit hemolysis during drawing of blood from a patient, comprising:
a catheter hub (52) having a proximal end (56) and a distal end (54);
a fluid collection device (40); and
the flow restriction device of any one of Claims 1 to 11, wherein the first lumen is in fluid communication with the catheter hub and the second lumen is in fluid communication with the fluid collection device.

## Patentansprüche

1. Durchflussbegrenzungsvorrichtung (100), umfassend: ein Gehäuse, das ein erstes Lumen (112), ein zweites Lumen (122) und einen zwischen dem ersten Lumen und dem zweiten Lumen angeordneten Hohlraum (116) definiert; und einen Einsatzkörper (130), der in dem Hohlraum angeordnet ist, wobei der Einsatzkörper umfasst: ein erstes Ende (134); ein zweites Ende (138); eine Längsachse, die sich durch das erste und zweite Ende erstreckt; eine Außenfläche; und einen Kanal (132), der auf der Außenfläche definiert ist, wobei sich der Kanal zwischen dem ersten und dem zweiten Ende erstreckt, wobei der Kanal einen ersten Abschnitt (136), der sich in einer ersten Richtung weg von der Längsachse erstreckt, wenn der Kanal von der Oberseite in einer Richtung senkrecht zur Längsachse gesehen wird, und einen zweiten Abschnitt (135) umfasst, der sich in einer zweiten Richtung zur Längsachse hin erstreckt, wenn der Kanal von oben in einer Richtung senkrecht zur Längsachse gesehen wird und der Kanal und eine innere Oberfläche des Hohlraums einen Fluiddurchgang in Fluidkommunikation mit dem ersten Lumen und dem zweiten Lumen definieren, **dadurch gekennzeichnet, dass** der zweite Abschnitt in einem Winkel zwischen 45 Grad und 90 Grad relativ zu dem ersten Abschnitt angeordnet ist.

2. Durchflussbegrenzungsvorrichtung nach Anspruch 1, wobei der Kanal einen Durchmesser von etwa 0,508 Millimetern bis etwa 0,762 Millimetern (etwa 0,02 Zoll bis etwa 0,03 Zoll) aufweist.

3. Durchflussbegrenzungsvorrichtung nach Anspruch 1, wobei der Kanal eine Länge von etwa 24,639 Millimetern bis etwa 33,02 Millimetern (etwa 0,97 Zoll bis etwa 1,30 Zoll) aufweist.

4. Durchflussbegrenzungsvorrichtung nach Anspruch 1, wobei der Fluiddurchgang so konfiguriert ist, den Durchflusswiderstand durch den Fluiddurchgang zu vergrößern, um die von der Fluidströmung erfahrenen Scherspannungen zu minimieren.

5. Durchflussbegrenzungsvorrichtung nach Anspruch 1, wobei der Kanal einen serpentinenförmigen Fluiddurchgang definiert.

6. Durchflussbegrenzungsvorrichtung nach Anspruch 1, wobei der Kanal zumindest teilweise sinusförmig ist.

7. Durchflussbegrenzungsvorrichtung nach Anspruch 1, wobei der Einsatzkörper einen zylindrischen Körper umfasst.

8. Durchflussbegrenzungsvorrichtung nach Anspruch 7, wobei sich der Kanal entlang der Außenfläche innerhalb eines Bogens eines Querschnitts des zylindrischen Körpers erstreckt.

9. Durchflussbegrenzungsvorrichtung nach Anspruch 8, wobei der Bogen etwa 180 Grad beträgt.

10. Durchflussbegrenzungsvorrichtung nach Anspruch 8, wobei der Bogen etwa 90 Grad beträgt.

11. Durchflussbegrenzungsvorrichtung nach Anspruch 1, wobei das Gehäuse einen männlichen Luer-Verbindungsabschnitt (110), der das erste Lumen definiert, und einen weiblichen Luer-Verbindungsabschnitt (120), der das zweite Lumen definiert, umfasst, und der männliche Luer-Verbindungsabschnitt und der weibliche Luer-Verbindungsabschnitt miteinander gekoppelt sind, um zusammenwirkend den Hohlraum zu definieren.

12. Durchflussbegrenzungsvorrichtung nach Anspruch 1, wobei das erste Lumen so konfiguriert ist, dass es mit einem Katheteransatzstück (52) verbunden werden kann.

13. Durchflussbegrenzungsvorrichtung nach Anspruch 1, wobei das zweite Lumen so konfiguriert ist, dass es mit einer Fluid-Sammelvorrichtung (40) gekoppelt werden kann.

14. Periphere intravenöse Katheterbaugruppe (10), die so konfiguriert ist, dass sie die Hämolyse während der Blutentnahme von einem Patienten begrenzt, umfassend:
ein Katheteransatzstück (52) mit einem proximalen Ende (56) und einem distalen Ende (54);
eine Fluid-Sammelvorrichtung (40); und
die Durchflussbegrenzungsvorrichtung nach einem der Ansprüche 1 bis 11, wobei das erste Lumen in Fluidkommunikation mit dem Katheteransatzstück steht und das zweite Lumen in Fluidkommunikation mit der Fluid-Sammelvorrichtung steht.

## Revendications

1. Dispositif de restriction du débit (100), comprenant: un boîtier définissant une première lumière (112), une deuxième lumière (122), et une cavité (116) disposée entre la première lumière et la deuxième lumière; et un corps d'insertion (130) disposé dans la cavité, le corps d'insertion comprenant: une première extrémité (134); une deuxième extrémité (138); un axe longitudinal s'étendant à travers la première et la deuxième extrémité; une surface extérieure;
un canal (132) défini sur la surface extérieure, dans lequel le canal s'étend entre la première et la deuxième extrémité, le canal comprenant une première partie (136) qui s'étend dans une première direction à l'écart de l'axe longitudinal, lorsque le canal est vu du dessus dans une direction perpendiculaire à l'axe longitudinal et une deuxième partie (135) qui s'étend dans une deuxième direction vers l'axe longitudinal, lorsque le canal est vu du dessus dans une direction perpendiculaire à l'axe longitudinal et le canal et une surface intérieure de la cavité définissent un passage de fluide en communication fluidique avec la première lumière et la deuxième lumière, **caractérisé en ce que** la
la deuxième partie est disposée à un angle compris entre 45 et 90 degrés par rapport à la première partie.

2. Le dispositif de restriction du débit de la revendication 1, dans lequel le canal a un diamètre d'environ 0,508 millimètre à environ 0,762 millimètre (environ 0,02 pouces à environ 0,03 pouces).

3. Le dispositif de restriction du débit de la revendication 1, dans lequel le canal a une longueur d'environ 24,639 millimètres à environ 33,02 millimètres (environ 0,97 pouces à environ 1,30 pouces).

4. Le dispositif de restriction du débit de la revendication 1, dans lequel le passage de fluide est configuré pour augmenter la résistance à l'écoulement à travers le passage de fluide afin de minimiser la contrainte de cisaillement subie par l'écoulement de fluide.

5. Le dispositif de restriction du débit de la revendication 1, dans lequel le canal définit un passage de fluide en forme de serpentin.

6. Le dispositif de restriction du débit de la revendication 1, dans lequel le canal est au moins partiellement sinusoïdal.

7. Le dispositif de restriction du débit de la revendication 1, dans lequel le corps de l'insertion comprend un corps cylindrique.

8. Le dispositif de restriction du débit de la revendication 7, dans lequel le canal s'étend le long de la surface extérieure à l'intérieur d'un arc de la section transversale du corps cylindrique.

9. Le dispositif de restriction du débit de la revendication 8, dans lequel l'arc est d'environ 180 degrés.

10. Le dispositif de restriction du débit de la revendication 8, dans lequel l'arc est d'environ 90 degrés.

11. Le dispositif de restriction du débit de la revendication 1, dans lequel le boîtier comprend une partie de connecteur luer mâle (110) définissant la première lumière et une partie de connecteur luer femelle (120) définissant la deuxième lumière, et la partie de connecteur luer mâle et la partie de connecteur luer femelle sont couplés ensemble pour définir la cavité de manière coopérative.

12. Le dispositif de restriction du débit de la revendication 1, dans lequel la première lumière est configurée pour être couplée à un embout de cathéter (52).

13. Le dispositif de restriction du débit de la revendication 1, dans lequel la deuxième lumière est configurée pour être couplée à un dispositif de collecte de fluide (40).

14. Ensemble de cathéter intraveineux périphérique (10) configuré pour limiter l'hémolyse pendant la prise de sang d'un patient, comprenant :
un embout de cathéter (52) ayant une extrémité proximale (56) et une extrémité distale (54)
un dispositif de collecte de fluide (40) ; et
le dispositif de restriction du débit de l'une des revendications 1 à 11, dans lequel la première lumière est en communication fluidique avec l'embout de cathéter et la deuxième lumière est en communication fluidique avec le dispositif de collecte de fluide.
